Europäisches Patentamt

European Patent Office

Office européen des brevets

(19)

(11) **EP 1 522 505 A2**

## (12) EUROPEAN PATENT APPLICATION

(43) Date of publication:
**13.04.2005 Bulletin 2005/15**

(51) Int Cl.⁷: **B65D 83/14**

(21) Application number: **04026926.8**

(22) Date of filing: **14.05.2002**

(84) Designated Contracting States:
**AT BE CH CY DE DK ES FI FR GB GR IE IT LI LU MC NL PT SE TR**

(30) Priority: **22.06.2001 US 300287 P**

(62) Document number(s) of the earlier application(s) in accordance with Art. 76 EPC:
**02726007.4 / 1 399 374**

(71) Applicant: **3M Innovative Properties Company St. Paul, MN 55133-3427 (US)**

(72) Inventors:
 • **Arsenault, Cathleen, M.**
 **Saint Paul MN 55133-3427 (US)**

 • **Castro, Gustavo, H.**
 **Saint Paul MN 55133-3427 (US)**
 • **Herdtle, Thomas**
 **Saint Paul MN 55133-3427 (US)**

(74) Representative: **VOSSIUS & PARTNER**
 **Siebertstrasse 4**
 **81675 München (DE)**

Remarks:
 This application was filed on 12 - 11 - 2004 as a divisional application to the application mentioned under INID code 62.

## (54) Metering valve for a metered dose inhaler

(57) A novel metering valve having improved flow for delivery of an aerosol formulation is disclosed. Methods of improving the flow characteristics of a metering valve are also disclosed.

**Fig. 2**

## Description

## Background

Metering valves are common means by which aerosols are dispensed from aerosol containers. Metering valves are particularly useful for administering medicinal formulations that include a liquefied gas propellant and are delivered to a patient in an aerosol.

When administering medicinal formulations, the proper, predetermined amount of the formulation in each successive dose must be dispensed to the patient. In this way, a dose of formulation sufficient to produce the desired physiological response is delivered to the patient. Any dispensing system must be able to dispense doses of the medicinal formulation accurately and reliably to help assure the safety and efficacy of the treatment.

Metering valves have been developed to provide control over the dispensing of medicinal aerosol formulations. A metering valve regulates the volume of a medicinal formulation passing from a container to a patient via a metering chamber. The metering chamber thus defines the maximum amount of the formulation that will be dispensed as the next dose. Reliable and controllable flow of the medicinal formulation into the metering chamber is therefore highly desirable.

In some metering valves, the metering chamber fills with the medicinal formulation prior to the patient actuating the valve stem and thereby releasing the dose: After dispensing one dose, the metering chamber is refilled with formulation so that it is ready to discharge the next dose. Consequently, the metering chamber is full of the formulation at all times except for the brief time during which the valve stem is depressed by the user to discharge a dose. Also, the passageways through which the bulk formulation must flow to reach the metering chamber are often narrow and tortuous. As a result, metering valves configured in this way have a number of disadvantages resulting in, for example, erratic dosing due to loss of prime.

In other metering valves, the metering chamber does not materialize unless and until the valve stem is actuated. Generally, such metering valves have a small annular gap between the external surface of the valve stem and the internal surface of a surrounding valve body, i.e., the tight region between the valve stem and the body wall in which the valve stem and the body wall are in close proximity. As the valve stem is actuated, formulation flows through an inlet and into the small annular gap and the nascent, transient metering chamber, the formation of which is described below.

Actuation of the valve stem in such a metering valve can be divided into a filling stage and a discharge stage. The filling stage begins as the valve stem is depressed during actuation. The action of depressing the valve stem causes the development of a transient metering chamber. As the valve stem is depressed, the transient metering chamber expands and formulation enters the metering chamber. As displacement of the valve stem continues, a stage is reached at which filling of the transient metering chamber stops. Eventually, displacement of the valve stem continues to the discharge stage, in which the metered formulation is discharged. In these valves, a single actuation thus causes rapid filling of the transient metering chamber followed by discharge of the formulation to the patient. Thus, the metered formulation does not reside for any appreciable amount of time in the metering chamber. Such metering valves may provide delivery of more consistent doses of formulation than may be provided by metering valves in which the metering chamber fills with formulation before the valve stem is actuated.

While a metering valve having a transient metering chamber provides advantages over other types of metering valves for the delivery of aerosol formulations, the flow of formulation from the container to the metering chamber still may be disrupted or impeded. When this happens, formulation may be delivered in inconsistent or inaccurate doses.

What is needed is a metering valve for a metered dose inhaler that improves flow of formulation into the metering chamber, thereby providing consistent, accurate, dosages of formulation.

## Summary

It has been determined that one cause of disrupted or impeded flow of formulation may be due to the design of the metering valve. The length of the annular gap between the valve stem and the valve body may impact the flow of formulation into the nascent metering chamber. The length of the annular gap may be defined by the boundaries of a tight region between the valve stem and the body wall, i.e., a region in which the valve stem and the valve body are in relatively close proximity to each other. Generally, a metering valve including a relatively longer annular gap may provide an increased risk of turbulence, recirculation or an increased drop in pressure in the flow of formulation into the metering chamber, any or all of which may disrupt or impede the flow of formulation. Conversely, a metering valve including a relatively shorter annular gap may provide improved flow of formulation.

Accordingly, the present invention provides an aerosol metering valve including a body wall defining an internal chamber and comprising a metering gasket and a diaphragm, the diaphragm having walls defining an aperture; a valve stem comprising a body portion and a stem portion, the body portion comprising a bottom edge and being positioned in the internal chamber of the metering valve, the stem portion comprising a discharge outlet and passing through the aperture in slidable, sealing engagement with the diaphragm; an annular gap comprising an inlet and having a length of about 3.2 mm or less, defined by a distance from the bottom edge of the body portion of the valve stem to the inlet; and a flow path providing at least transient fluid communication between the internal chamber and the annular gap through the inlet.

In another aspect, the present invention provides an aerosol metering valve including a body wall defining an internal chamber and comprising a metering gasket and a diaphragm, the diaphragm having walls defining an aperture; a valve stem comprising a body portion and a stem portion, the body portion comprising a bottom edge and being positioned in the internal chamber of the metering valve, the stem portion comprising a discharge outlet and passing through the aperture in slidable, sealing engagement with the diaphragm; an annular gap comprising an inlet and having a width defined by a distance from the body wall to the valve stem, and also having a length defined by a distance from the inlet to the bottom edge of the body portion of the valve stem; and a flow path providing at least transient fluid communication between the internal chamber and the annular gap through the inlet; wherein a ratio of the length of the annular gap to the width of the annular gap is less than about 40:1.

In another aspect, the present invention provides a method of improving flow of an aerosol formulation in a metering valve. The method includes: a) providing a metering valve comprising i) a body wall defining an internal chamber and comprising a metering gasket and a diaphragm, the diaphragm having walls defining an aperture, ii) a valve stem comprising a body portion and a stem portion, the body portion comprising a bottom edge and being positioned in the internal chamber of the metering valve, the stem portion comprising a discharge outlet and passing through the aperture in slidable, sealing engagement with the diaphragm, iii) an annular gap comprising an inlet and having an original length defined by a distance from the inlet to the bottom edge of the body portion of the valve stem, and iv) a flow path providing at least transient fluid communication between the internal chamber.and the annular gap; and b) reconfiguring the inlet, the bottom edge of the body portion of the valve stem, or both so that a new length of the annular gap is defined by a distance from the inlet to the bottom edge of the body portion of the valve stem after the reconfiguring; wherein the new length of the annular gap is less than the original length of the annular gap.

In another aspect, the present invention provides method of improving flow of aerosol formulation in a metering valve. The method includes: a) providing a metering valve comprising i) a body wall defining an internal chamber and comprising a metering gasket and a diaphragm, the diaphragm having walls defining an aperture, ii) a valve stem comprising a body portion and a stem portion, the body portion comprising a bottom edge and being positioned in the internal chamber of the metering valve in slidable, sealing engagement with the metering gasket, the stem portion comprising a discharge outlet and passing through the aperture in slidable, sealing engagement with the diaphragm, iii) an annular gap comprising an inlet and having a length defined by a distance from the inlet to the bottom edge of the body portion of the valve stem, and iv) a flow path providing at least transient fluid communication between the internal chamber and the annular gap through the inlet; b) reconfiguring the metering valve to change one or more variables in the formula:

$$P= \frac{6LQ\mu}{\pi fh^3R} + \frac{(1+K)Q^2\rho}{8\pi^2f^2h^2R^2},$$

wherein:

P = pressure drop,
L = length of the annulus [m],
Q = volumetric flow rate [$m^3$/s],
$\mu$ = fluid viscosity [kg/m·s],
f = average annulus utilization [0-1],
h = distance between the inner and outer wall of the annulus [m],
R = inner radius of the annulus [m],
$\rho$ = density of the formulation [kg/$m^3$], and
K = entrance loss coefficient,

so that flow of formulation is improved.

Further preferred embodiments are described as follows:

1. An aerosol metering valve comprising:

a body wall defining an internal chamber and comprising a metering gasket and a diaphragm, the diaphragm having walls defining an aperture;
a valve stem comprising a body portion and a stem portion, the body portion comprising a bottom edge and being positioned in the internal chamber of the metering valve, the stem portion comprising a discharge outlet and passing through the aperture in slidable, sealing engagement with the diaphragm;
an annular gap comprising an inlet and having a length of about 3.2 mm or less, wherein the length of the annular gap is defined by the distance from the bottom edge of the body portion of the valve stem to the inlet; and
a flow path providing at least transient fluid communication between the internal chamber and the annular gap through the inlet.

2. The aerosol metering valve of embodiment 1 wherein the flow path comprises a passageway through the body portion of the valve stem.

3. The aerosol metering valve of embodiment 1 wherein the flow path comprises a clearance between the body portion of the valve stem and the metering gasket.

4. The aerosol valve of embodiment 1 wherein the length of the annular gap is about 2.0 mm or less.

5. The aerosol valve of embodiment 1 wherein the length of the annular gap is about 1.0 mm or less.

6. The aerosol valve of embodiment 1 wherein the length of the annular gap is about 0.4 mm or less.

7. The aerosol valve of embodiment 1 wherein the length of the annular gap is about 0.1 mm or less.

8. An aerosol metering valve comprising:

> a body wall defining an internal chamber and comprising a metering gasket and a diaphragm, the diaphragm having walls defining an aperture;
> a valve stem comprising a body portion and a stem portion, the body portion comprising a bottom edge and being positioned in the internal chamber of the metering valve, the stem portion comprising a discharge outlet and passing through the aperture in slidable, sealing engagement with the diaphragm;
> an annular gap comprising an inlet and having a width defined by the distance from the body wall to the valve stem, and also having a length defined by the distance from the inlet to the bottom edge of the body portion of the valve stem, and wherein ratio of the length of the annular gap to the width of the annular gap is less than about 40:1; and a flow path providing at least transient fluid communication between the internal chamber and the annular gap through the inlet.

9. The aerosol metering valve of embodiment 8 wherein the distance from the body wall to the valve stem is substantially uniform, and wherein the width of the annular gap equals the distance from the body wall to the valve stem.

10. The aerosol metering valve of embodiment 8 wherein the distance from the body wall to the valve stem is non-uniform and comprises a maximum distance, and wherein the width of the annular gap equals the maximum distance.

11. The aerosol metering valve of embodiment 8 wherein the flow path comprises a passageway through the body portion of the valve stem.

12. The aerosol metering valve of embodiment 8 wherein the flow path comprises a clearance between the body portion of the valve stem and the metering gasket.

13. The aerosol metering valve of embodiment 8 wherein the ratio of the length of the annular gap to the width of the annular gap is less than about 25:1.

14. The aerosol metering valve of embodiment 8 wherein the ratio of the length of the annular gap to the width of the annular gap is less than about 20:1.

15. The aerosol metering valve of embodiment 8 wherein the ratio of the length of the annular gap to the width of the annular gap is less than about 12.5: 1.

16. The aerosol metering valve of embodiment 8 wherein the ratio of the length of the annular gap to the width of the annular gap is less than about 5:1

17. The aerosol metering valve of embodiment 8 wherein the ratio of the length of the annular gap to the width of the annular gap is less than about 2:1.

18. The aerosol metering valve of embodiment 8 wherein the ratio of the length of the annular gap to the width of the annular gap is less than about 1:1.

19. An aerosol metering valve comprising:

> a body wall defining an internal chamber and comprising a metering gasket and a diaphragm, the diaphragm having walls defining an aperture;
> a valve stem comprising a body portion and a stem portion, the body portion comprising a bottom edge and being positioned in the internal chamber of the metering valve, the stem portion comprising a discharge outlet and passing through the aperture in slidable, sealing engagement with the diaphragm;
> an annular gap comprising an inlet and having a width defined by the distance from the body wall to the valve stem, and also having a length of about 3.2 mm or less, defined by the distance from the inlet to the bottom edge of the body portion of the valve stem,

wherein a ratio of the length of the annular gap to the width of the annular gap is less than about 320: 1; and a flow path providing at least transient fluid communication between the internal chamber and the annular gap through the inlet.

20. The aerosol metering valve of embodiment 19 wherein the distance from the body wall to the valve stem is substantially uniform, and wherein the width of the annular gap equals the distance from the body wall to the valve stem.

21. The aerosol metering valve of embodiment 19 wherein the distance from the body wall to the valve stem is non-uniform and comprises a maximum distance, and wherein the width of the annular gap equals the maximum distance.

22. The aerosol metering valve of embodiment 19 wherein the flow path comprises a passageway through the body portion of the valve stem.

23. The aerosol metering valve of embodiment 19 wherein the flow path comprises a clearance between the body portion of the valve stem and the metering gasket.

24. The aerosol metering valve of embodiment 19 wherein the ratio of the length of the annular gap to the annular gap is less than about 200:1.

25. The aerosol metering valve of embodiment 19 wherein the ratio of the length of the annular gap to the width of the annular gap is less than about 128:1.

26. The aerosol metering valve of embodiment 19 wherein the ratio of the length of the annular gap to the width of the annular gap is less than about 100:1.

27. The aerosol metering valve of embodiment 19 wherein the ratio of the length of the annular gap to the width of the annular gap is less than about 80:1.

28. The aerosol metering valve of embodiment 19 wherein the ratio of the length of the annular gap to the width of the annular gap is less than about 64:1.

29. A method of improving flow of an aerosol formulation in a metering valve comprising:

a) providing a metering valve comprising

i) a body wall defining an internal chamber and comprising a metering gasket and a diaphragm, the diaphragm having walls defining an aperture,
ii) a valve stem comprising a body portion and a stem portion, the body portion comprising a bottom edge and being positioned in the internal chamber of the metering valve, the stem portion comprising a discharge outlet and passing through the aperture in slidable, sealing engagement with the diaphragm,
iii) an annular gap comprising an inlet and having an original length defined by the distance from the inlet to the bottom edge of the body portion of the valve stem, and
iv) a flow path providing at least transient fluid communication between the internal chamber and the annular gap; and

b) reconfiguring the inlet, the bottom edge of the body portion of the valve stem, or both so that a new length of the annular gap is defined by the distance from the inlet to the bottom edge of the body portion of the valve stem after the reconfiguring;

wherein the new length of the annular gap is less than the original length of the annular gap.

30. The method of embodiment 29 wherein the flow path comprises a passageway through the body portion of the valve stem.

31. The method of embodiment 29 wherein the flow path comprises a clearance between the body portion of the valve stem and the metering gasket.

32. A method of improving flow of aerosol formulation in a metering valve comprising:

a) providing a metering valve comprising

i) a body wall defining an internal chamber and comprising a metering gasket and a diaphragm, the diaphragm having walls defining an aperture,
ii) a valve stem comprising a body portion and a stem portion, the body portion comprising a bottom edge and being positioned in the internal chamber of the metering valve in slidable, sealing engagement with the metering gasket, the stem portion comprising a discharge outlet and passing through the aperture in slidable, sealing engagement with the diaphragm,
iii) an annular gap comprising an inlet and having a length defined by the distance from the inlet to the bottom edge of the body portion of the valve stem, and
iv) a flow path providing at least transient fluid communication between the internal chamber and the annular gap through the inlet;

b) reconfiguring the metering valve to change one or more variables in the formula:

$$P = \frac{6LQ\mu}{\pi f h^3 R} + \frac{(1+K)Q^2\rho}{8\pi^2 f^2 h^2 R^2},$$

wherein:

P = pressure drop,
L = length of the annulus [m],
Q = volumetric flow rate [m³/s],
$\mu$ = fluid viscosity [kg/m·s],
f = average annulus utilization [0-1],
h = distance between the inner and outer wall of the annulus [m],
R = inner radius of the annulus [m],
$\rho$ = density of the formulation [kg/m³], and
K = entrance loss coefficient,

so that P is decreased.

33. The method of embodiment 32 wherein the flow path comprises a passageway through the body portion of the valve stem.

34. The method of embodiment 32 wherein the flow path comprises a clearance between the body portion of the valve stem and the metering gasket.

**Brief Description of the Drawings**

FIG. 1 is a cross-sectional view of a metered dose inhaler including aerosol metering valve.
FIG. 2 is an enlarged cross-sectional view of an aerosol metering valve in the resting position.
FIG. 3 is a further enlarged cross-sectional view of the aerosol metering valve of FIG. 2, illustrating one embodiment of the present invention.
FIG. 4 is an enlarged cross-sectional view of an alternative aerosol metering valve in the resting position.
FIG. 5 is a further enlarged cross-sectional view of the aerosol metering valve of FIG. 4, illustrating one embodiment of the present invention.
FIG. 6 is an enlarged cross-sectional view of another alternative aerosol metering valve in the resting position.
FIG. 7a is a further enlarged cross-sectional view of the aerosol metering valve of FIG. 6 in the resting position, illustrating one embodiment of the present invention.
FIG. 7b is a further enlarged cross-sectional view of the aerosol metering valve of FIG. 6 in the filled stage of operation, illustrating one embodiment of the present invention.
FIG. 8 is an enlarged cross-sectional view of an aerosol metering valve in the filling stage of operation.
FIG. 9 is an enlarged cross-sectional view of an aerosol metering valve in the filled stage of operation.
FIG. 10 is an enlarged cross-sectional view of an aerosol metering valve in the discharging stage of operation.

**Detailed Description of Illustrative Embodiments of the Invention**

The present invention relates to an aerosol metering valve having improved flow characteristics. The metering valve of the present invention includes an annular gap having a relatively short length. The present invention also relates to improving the flow characteristics of a metering valve by reconfiguring the metering valve so that the annular gap has a shorter length than was present in an original configuration of the metering valve.

The following description is set forth in terms of an aerosol metering valve used to dispense an aerosol formulation from an aerosol container. However, the metering valve and methods of the present invention have application to delivery of virtually any pressurized fluid requiring an accurate, metered dose. In particular, the metering valve described herein is useful for dispensing medicinal aerosol formulations.

When used to dispense a medicinal aerosol formulation, the metering valve of the present invention may be used to administer virtually any aerosol formulation of drug into a body cavity of a patient, such as the mouth, nose, anus, vagina, ears, or onto the eyes or any skin area of the patient. However, the present invention is not limited to medicinal applications and may be used wherever a precise amount of material from a pressurized fluid is to be delivered to a given region.

Various configurations of metering valves are known. Despite differences in metering valve configuration, there are many similarities in the general structure, function, and operation of metering valves that make the features of the present invention generally applicable to many metering valve designs. While the description of the invention that follows is provided in the context of a sampling of different metering valve designs, the principles embodied in the present invention are applicable to other metering valve designs as well. Consequently, the following description of the present invention should be considered illustrative and should not be construed to unduly limit the scope of the present invention.

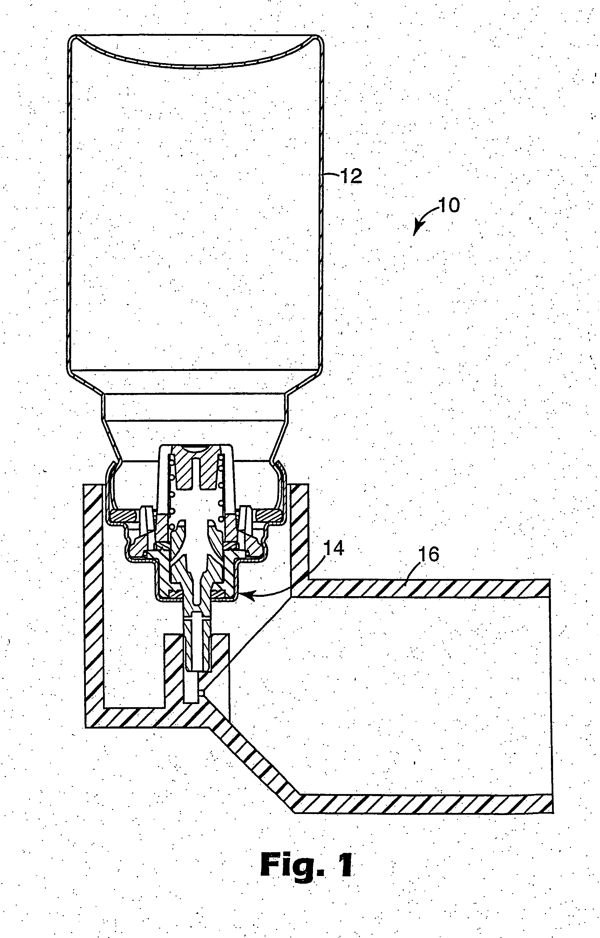

Referring to FIG. 1, an aerosol dispensing apparatus, generally designated as **10**, is illustrated incorporating one metering valve design. The top end of the metering valve **14** is crimped around the end of a conventional aerosol container **12**, while a conventional discharge piece **16** is mounted around the bottom of the metering valve **14.** Thus, aerosol formulation is dispensed downwardly from the aerosol container **12,** through the metering valve **14,** then through the discharge piece **16** where it is delivered to a patient. The discharge piece **16** directs the aerosol formulation toward the body cavity or skin area to which the formulation is to be delivered. For example, discharge piece **16** may be a mouthpiece that can be inserted into the patient's mouth, thereby'providing oral administration of the aerosol formulation.

The aerosol dispensing device shown in FIG. 1 is merely one example of how the metering valve can be incorporated into a dispensing apparatus. Furthermore, the configuration of the discharge piece **16** depends upon the application for the aerosol.

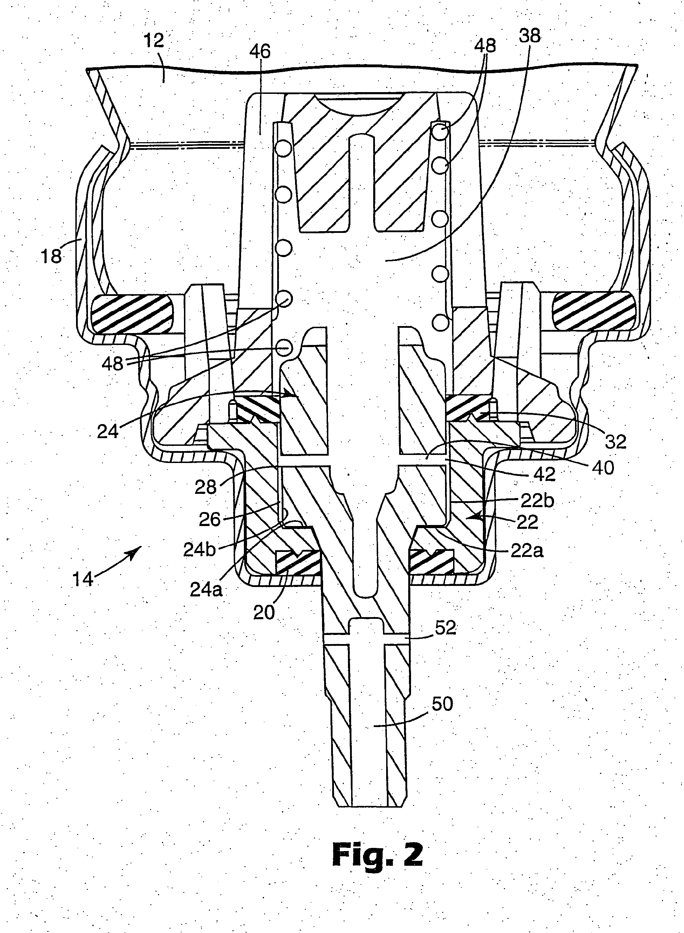

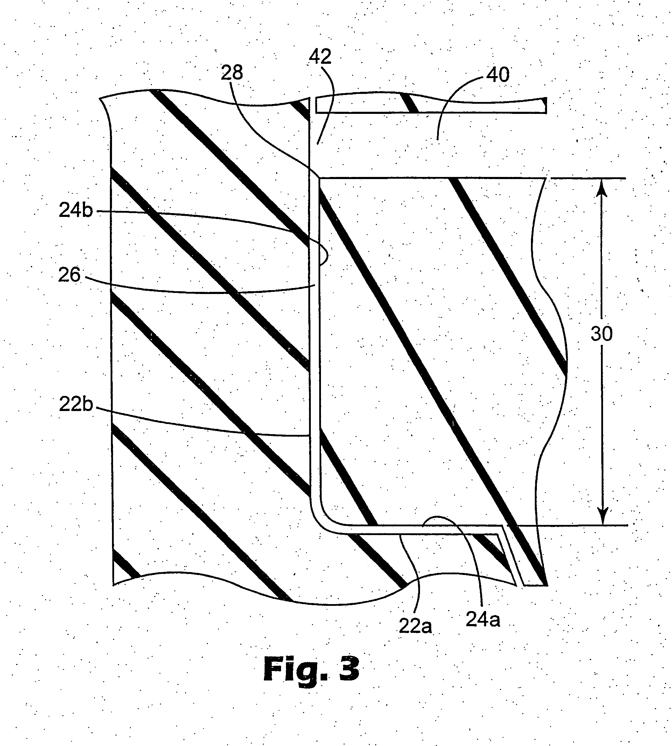

In each of FIGS. 2, 4 and 6, a metering valve design is shown in isolation for ease of illustration. While neither the aerosol container **12** nor the discharge piece **16** are completely shown in any of FIGS. 2, 4 or 6, it should be understood that the metering valve shown in each of these figures may be combined with an aerosol container **12,** discharge piece **16**, or both, as shown in FIG. 1. Each of FIGS. 3,5 and 7a shows an expanded view of the metering valve design illustrated in FIGS. 2, 4 and 6, respectively. FIGS. 3, 5 and 7a illustrate the features of the present invention as applied to the metering valve designs shown in FIGS. 2, 4 and 6. It should be noted that the dimensions of the annular gaps illustrated in the figures are not intended to be drawn to scale. Specifically, for ease of illustration, the annular gaps are drawn to be larger in relation to the rest of the metering valve, in both length and width, than they may be in certain embodiments of the present invention.

Referring to FIG. 2, 4 and 6, a different metering valve design is shown in a resting position in each figure. Each metering valve **14** generally includes a housing **18** that serves to house the various components of the metering valve **14**. The top portion of the housing **18** attaches to the aerosol container **12** (as shown in FIG.1). A valve body **22** is seated within the valve housing **18** and in turn provides a housing for a valve stem **24**. The valve body may include a floor **22a** and a wall **22b**. The housing **18,** the valve body **22** and a diaphragm **20** may be aligned so that, together, they form an aperture.

The metering valve **14** includes an interior chamber **38,** a portion of which is occupied by a valve stem **24**. One or more ports **46** provide fluid communication between the interior chamber 38 and the aerosol container 12. A spring 48 may serve to bias the valve stem **24** toward the resting position. However, any suitable means for biasing the valve stem **24** into the resting position may be used. Alternatively, the valve stem **24** may be unbiased.

The valve stem **24** generally includes a body portion including a bottom edge **24a** and a side wall **24b**. A stem portion of the valve stem **24** extends through the aperture and is in slidable, sealing contact with the diaphragm **20**. The stem portion of the valve stem **24** may include a discharge outlet **50** through which a metered dose of formulation may be discharged. The discharge outlet **50** may include one or more side holes **52**.

The body portion of the valve stem **24** may be generally configured to have substantially the same shape as, but to be slightly smaller than, the surrounding wall of the valve body **22b**. Thus, an annular gap **26** is formed between the valve body wall **22b** and the side wall of the valve stem **24b**. In certain metering valve designs in which the body portion of the valve stem **24** and the valve body wall **22b** are both circular in cross-section, the annular gap **26** will form a ring in cross-section. However, the body portion of the valve stem **24** and valve body wall **22b**, and therefore the annular gap **26,** may be any suitable shape.

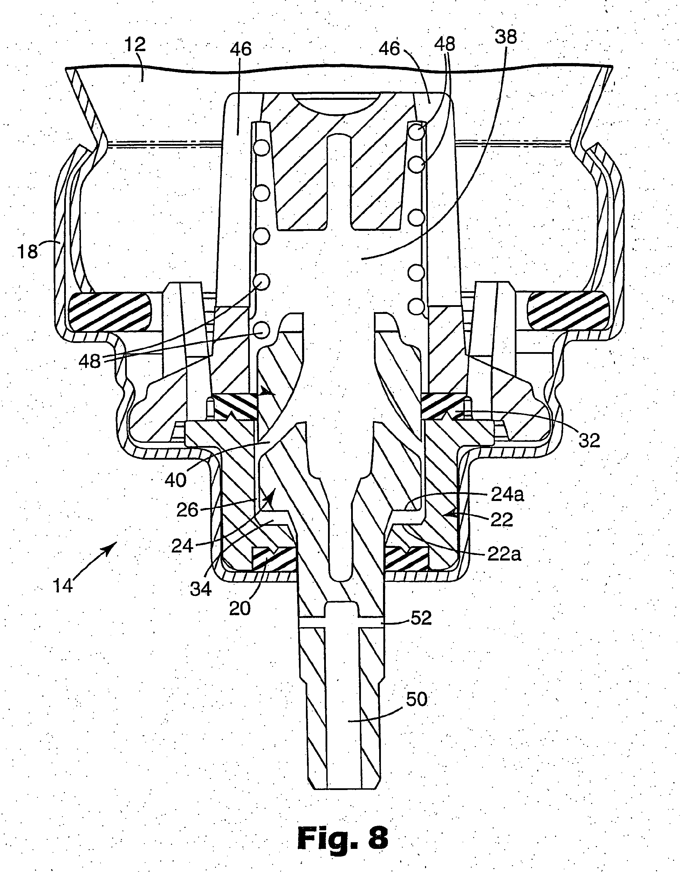

In the resting position shown in FIGS. 2, 4 and 6, the body portion of the valve stem **24** fits concentrically inside the valve body **22** and provides sufficient clearance for the annular gap **26**. When the valve stem **24** is actuated, the valve stem **24** is displaced into the interior chamber **38** of the metering valve **14** and a space is created between the bottom edge of the body portion of the valve stem **24a** and the floor of the valve body **22a**. The space thus created forms the major part of the metering chamber **34**, shown in FIG. 8 and discussed in more detail below. Because of the configuration of the body portion of the valve stem **24** and the valve body **22,** only a small percentage of the metering chamber volume, that represented by the volume of the annular gap **26,** is present when the metering valve **14** is in the resting position.

In each of the metering valve designs, the metering valve **14** also includes at least two annular gaskets: the diaphragm **20** and the metering gasket **32**. The diaphragm **20** isolates the formulation in the aerosol container **12** from the exterior of the valve by forming three fluid tight seals: 1) an annular seal between the diaphragm **20** and the valve stem **24** where the valve stem extends out of the valve housing, 2) a compressive planar or face seal between the diaphragm **20** and the housing **18**, and 3) a compressive planar or face seal between the diaphragm **20** and the valve body **22**. The metering gasket **32** transiently isolates the formulation in the metering chamber **34** from the aerosol container **12** by forming two fluid tight seals: 1) an annular seal between the metering gasket **32** and the body portion of the valve stem **24**, and 2) a compressive planar or face seal between the metering gasket **32** and the valve body **22**. In this way, the metering gasket **32** provides a means for terminating the flow of formulation from the aerosol container **12** to the metering chamber **34** during actuation of the valve stem **24.**

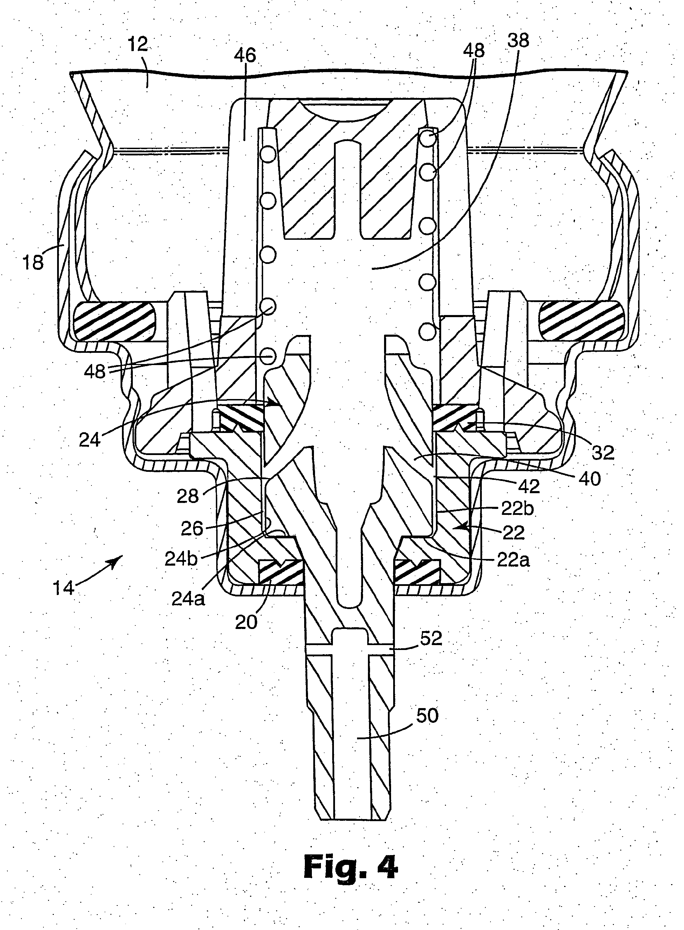

A flow path provides at least transient fluid communication between the interior chamber **38** and the annular gap **26** through an inlet **28.** In some metering valve designs, shown in FIGS. 2 and 4, the flow path may include a passage **40** through the interior of the body portion of the valve stem **24**. In such metering valves, the passage **40** through the interior of the body portion of the valve stem **24** provides fluid communication between the interior chamber **38** and the annular gap **26** through an opening **42**. As the valve stem **24** is actuated, it is displaced into the interior chamber **38**. Eventually, the extent of this displacement may be sufficient to cause the opening **42** to begin to be occluded by the metering gasket **32**. Further actuation of the valve stem **24** causes the portion of the valve stem side wall

**24b** between the opening **42** and the stem portion of the valve stem **24** to come into sealing engagement with the metering gasket **32**. At this point, the metering gasket **32** obstructs the flow path that provides fluid communication between the interior chamber **38** and the annular gap **26,** thereby terminating the flow of formulation into the metering chamber **34** and isolating the metering chamber **34** from the aerosol container **12**. Because the metering gasket **32** is in sealing engagement with the valve stem **24,** the metering gasket **32** maintains the isolation of the metering chamber **34** from the aerosol container **12** as the valve stem **24** is actuated further, thereby preventing additional flow of formulation into the metering chamber **34**.

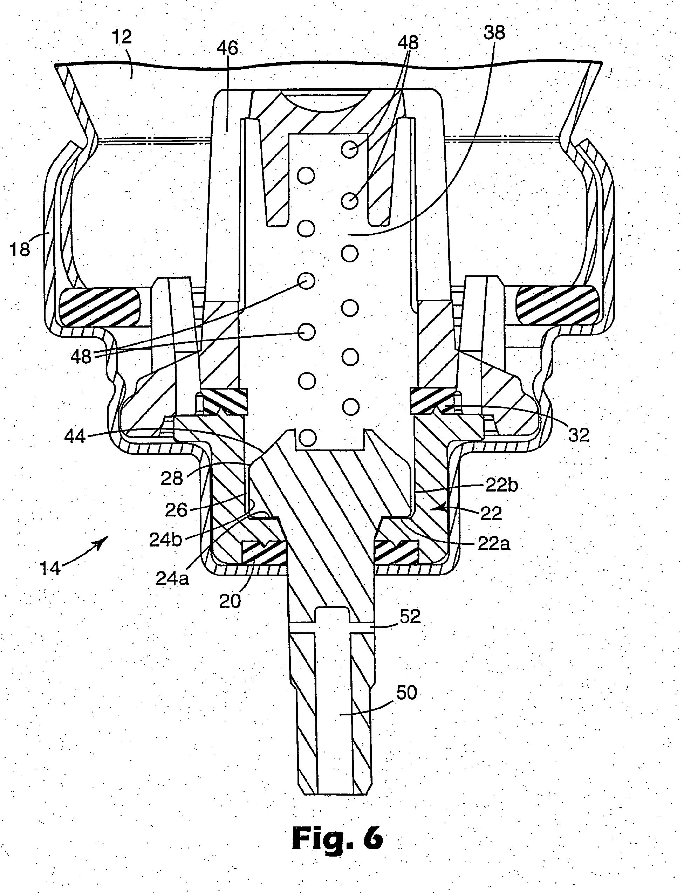

In another design, shown in FIG. 6, the flow path may exist between the metering gasket **32** and the body portion of the valve stem **24**. As the valve stem **24** is actuated, it is displaced into the interior chamber **38**. Eventually, the extent of this displacement will be sufficient to cause a sealing surface **44** on the valve stem **24** to contact the metering gasket 32, thereby forming a seal between the metering gasket 32 and the valve stem 24. This seal obstructs the flow path that provides fluid communication between the interior chamber **38** and the annular gap **26**, thereby terminating the flow of formulation from the aerosol container **12** to the metering chamber **34**. As the valve stem **24** continues to be displaced, it remains in continuous sealing contact with the metering gasket **32**, thereby preventing additional flow of formulation from the aerosol container **12** to the metering chamber **34**.

The annular gap **26** is shown in greater detail in FIGS. 3, 5, 7a and 7b. The annular gap **26** is defined by the space bounded by the side wall of the valve body **24b**, the wall of the valve stem **22b**, an inlet **28** and a line extending parallel to the horizontal axis of the valve stem from the bottom edge of the valve stem **24a**. The distance between the inlet **28** and bottom edge of the valve stem **24a**, defines the length of the annular gap **30**. In the resting position, the bottom edge of the valve stem **24a** is substantially in contact with the floor of the valve body **22a**. Generally, the inlet **28** is defined as the point along the flow path where the formulation first enters the tight region between the side wall of the valve stem **24b** and the wall of the valve body **22b**. Because different metering valve designs employ a variety of structures and valve stem configurations, the particular structures used to define the position of the inlet may vary between different metering valve designs. The inlet **28** is defined for a sample of exemplary metering valve designs in the following description. However, the inlet **28** may be defined for any metering valve design by reference to the tight region between the side wall of the valve stem **24b** and the wall of the valve body **22b**.

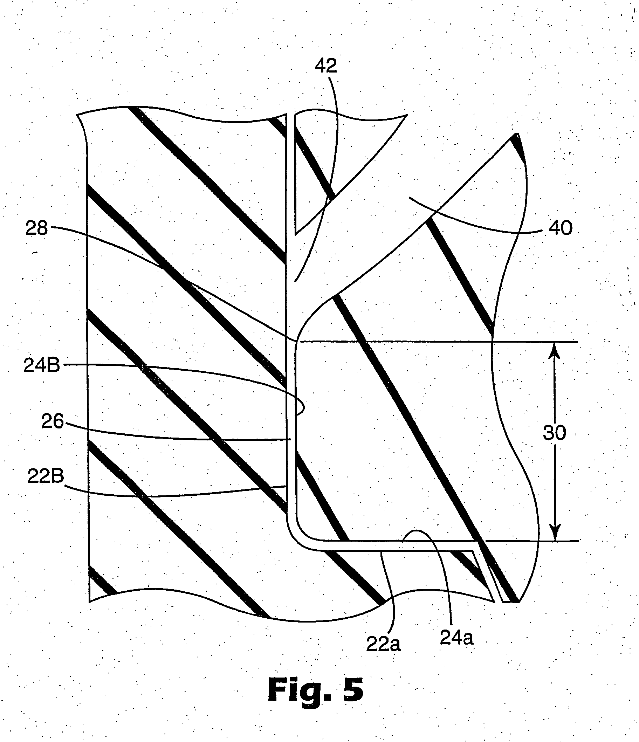

FIGS. 3 and 5 show the configuration of the annular gap **26** in a metering valve designed so that the flow path proceeds through the interior of the valve stem **24.** Formulation flows through the passage **40** and into the annular gap **26** through an inlet **28**. The inlet **28** is substantially parallel to the horizontal axis of the valve stem from a point at the bottom edge of the passage opening **42.** If the bottom edge of the passage opening **42** forms a corner, the inlet **28** is located at the edge of the corner, as shown in FIG. 3. If the bottom edge of the passage opening **42** is rounded, the inlet **28** is located at the point at which the tangent of the curve defined by the rounding is substantially collinear with the side wall of the valve body **24b**, as shown in FIG. 5. If the bottom edge of the passage opening **42** is beveled, the inlet **28** is located at the passage opening **42** corner, one side of which includes the side wall of the valve body **24b**. The length of the annular gap **30** is illustrated for each of the embodiments of the present invention shown in FIGS. 3 and 5.

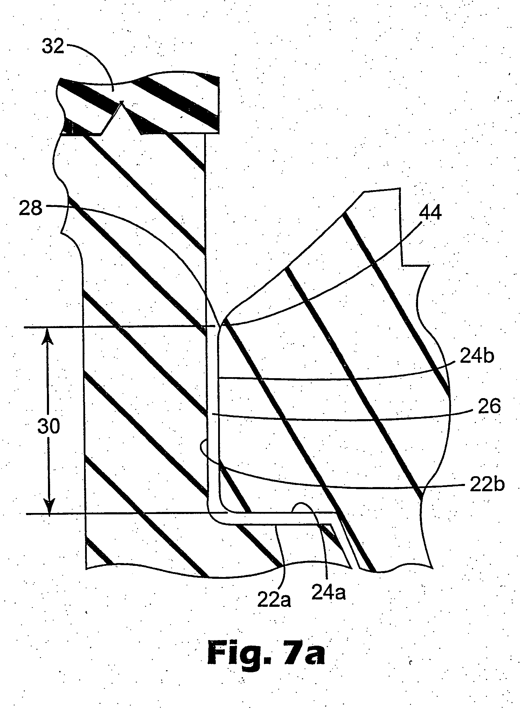

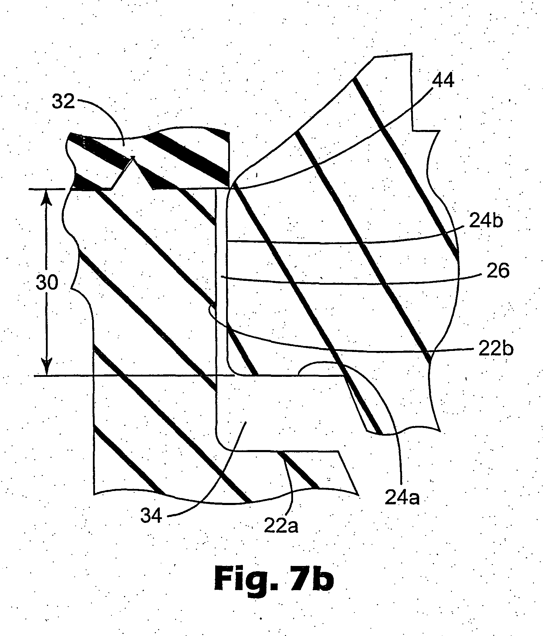

FIGS. 7a and 7b show the configuration of the annular gap **26** in a metering valve designed so that the flow path proceeds between the metering gasket **32** and the body portion of the valve stem **24**. In such a metering valve, the inlet **28** is located at the point of the sealing surface **44** that first makes contact with the metering gasket **32,** as shown in FIG. 7b. The length of the annular gap **30** is illustrated in each of FIGS. 7a and 7b.

As described in more detail below, the dimensions of the annular gap **26** may be designed to provide suitable performance of the metering valve **14** for delivery of a particular formulation. For example, the dimensions of the annular gap **26** may impact the flow of formulation from the aerosol container **12** into the metering chamber **34**. Additionally, the dimensions of the annular gap **26** determine the volume of the annular gap and, therefore, the volume of formulation that may be present in the annular gap **26** prior to actuation of the valve stem **24.**

One measure of the quality of flow of formulation in a metering valve is the pressure drop experienced by the formulation as it flows from the aerosol container **12** to the metering chamber **34**. As used herein, pressure drop means an energy loss per unit volume. The energy loss is expressed in terms of an equivalent pressure difference between two points in the flow field. For example, pressure drop may exist in a flow path where a fluid is forced to turn sharply at relatively high speed. If the pressure drop is of sufficient magnitude, the formulation may be subject to cavitation, leading to inaccurate or ineffective dosing. Cavitation of the formulation is thus undesirable. Consequently, it may be desirable to be able to design a metering valve that has improved flow characteristics so that the risk of cavitation of the formulation is reduced. One way to accomplish this objective is to design a metering valve so that the pressure drop experienced by the formulation is reduced.

The relationship between pressure drop and the length of the annular gap may be determined to a first order of approximation according to Formula I, provided below. Formula I assumes laminar flow through a uniform cylindrical annular gap with a valve stem radius significantly larger than the width of the annular gap. Moreover, Formula I applies to the flow of formulation in various designs of metering valves so long as the metering valve design includes an annular gap.

$$P = \frac{6LQ\mu}{\pi f h^3 R} + \frac{(1+K)Q^2\rho}{8\pi^2 f^2 h^2 R^2},\qquad \text{Formula I}$$

wherein:

P = pressure drop,
L = length of the annular gap [m],
Q = volumetric flow rate [m³/s],
μ = fluid viscosity [kg/m·s],
f = average annular gap utilization [0-1],
h = width of the annular gap[m],
R = inner radius of the annular gap (or radius of the valve stem) [m],
ρ = density of the formulation [kg/m³], and
K = entrance loss coefficient.

Thus, with all other variables held constant, pressure drop varies according to the length of the annular gap. In fact, if the viscous contribution to pressure drop,

$$P_{visc} = \frac{6LQ\mu}{\pi f h^3 R},$$

is much greater than the kinetic energy contribution to pressure drop,

$$P_{KE} = \frac{(1+K)Q^2\rho}{8\pi^2 f^2 h^2 R^2},$$

such as may occur when the formulation is very viscous, then total pressure drop, P, varies almost in direct proportion to the length of the annular gap.

Therefore, designing a metering valve with a minimal annular gap length minimizes the pressure drop and, therefore, reduces the likelihood and extent of cavitation of the formulation.

As stated above, Formula I assumes a uniform cylindrical annular gap in which the width of the annular gap (h) is constant. However, the principles of the present invention apply equally to metering valves in which the width of the annular gap varies. For example, a non-uniform annular gap width may be apparent along the length of the gap in a vertical cross-section. Alternatively, a non-uniform annular gap width may be apparent radially in a horizontal cross-section. For the purposes of approximating the pressure drop (P) according to Formula I, the width of the annular gap (h) for a metering valve having a non-uniform annular gap width is accounted for by integrating differential forms of the formula over the non-uniformity. For example, the pressure drop in a metering valve having a non-uniform annular gap width that is apparent in vertical cross-section may be approximated by integrating differential forms of Formula I along the length of the annular gap. Alternatively, the pressure drop in a metering valve having a non-uniform annular gap width that is apparent in horizontal cross-section may be approximated by integrating differential forms of Formula I azimuthally. Regardless of the particular nature of the non-uniform annular gap, pressure drop is generally reduced if the length of the annular gap (L) is shortened according to Formula I. Consequently, the flow of formulation is improved if the length of the annular gap is shortened regardless of any variation in the width of the annular gap.

For purposes not directly related to approximating pressure drop by using Formula I, such as computing a ratio of the length of the annular gap to the width of the annular gap, the width of a non-uniform annular gap may be approximated by selecting a representative annular gap width. For such purposes, one may select a maximum width, a minimum width or an average width of the non-uniform annular gap as a representative annular gap width.

In accordance with the principles described above, one embodiment of the present invention includes an annular gap having a length of about 3.2 mm. Various alternative embodiments include an annular gap having a length of about 2.0 mm, about 1.0 mm, about 0.4 or about 0.1 mm. Certain embodiments of the present invention include an annular gap having a width of about 0.08 mm. Various alternative embodiments include an annular gap having a width of about 0.1 mm, about 0.05 mm, about 0.025 mm, or about 0.01 mm. Greater widths, such as about 0.2 mm, about 0.5 mm or 1.0 mm, are also possible if desired for a particular application,

**Operation of the metering valve**

The various metering valve designs described above operate in a similar manner to one another. Between doses, each metering valve design described above exists in a resting position. Actuation of the metering valve involves the valve stem passing sequentially through at least three stages of operation in order to dispense a dose of formulation: a filling stage, a filled stage and a discharging stage. After dispensing a dose of formulation, the metering valve returns to the resting position. The operation of the metering valves will be described below in greater detail with some description provided for the differences in operation resulting from differences in configuration of the various metering valve designs. However, the principles of the present invention are equally applicable to all of the metering valve designs described, as well as to designs not described, herein.

In the filling stage, shown in FIG. 8, the valve stem **24** is displaced inwardly into the interior chamber **38.** As the valve stem **24** is displaced inwardly, the metering chamber **34** is formed between the floor of the valve body **22a** and the bottom edge of the valve stem **24a**. The volume of the metering chamber **34** increases as the valve stem is displaced.

The aerosol formulation enters the filling volume of the metering chamber **34** in the following manner. Formulation from the aerosol container **12** passes through the one or more metering valve ports **46** and into the interior chamber **38** of the metering valve. From the interior chamber **38,** the formulation follows the flow path to the annular gap **26.** In the metering valve design shown in FIGS. 2-5, the flow path follows the passage **40** through the interior of the valve stem. In the metering valve design shown in FIGS. 6, 7a and 7b, the flow path proceeds between the metering gasket **32** and the valve stem **24.** In either design, as the valve stem **24** is moved from the resting position into the filling stage, aerosol formulation passes from the aerosol container **12** to the metering chamber **34** immediately upon actuation of the valve stem **24.** Formulation continues to fill the metering chamber **34** until the metering valve **14** reaches the filled stage.

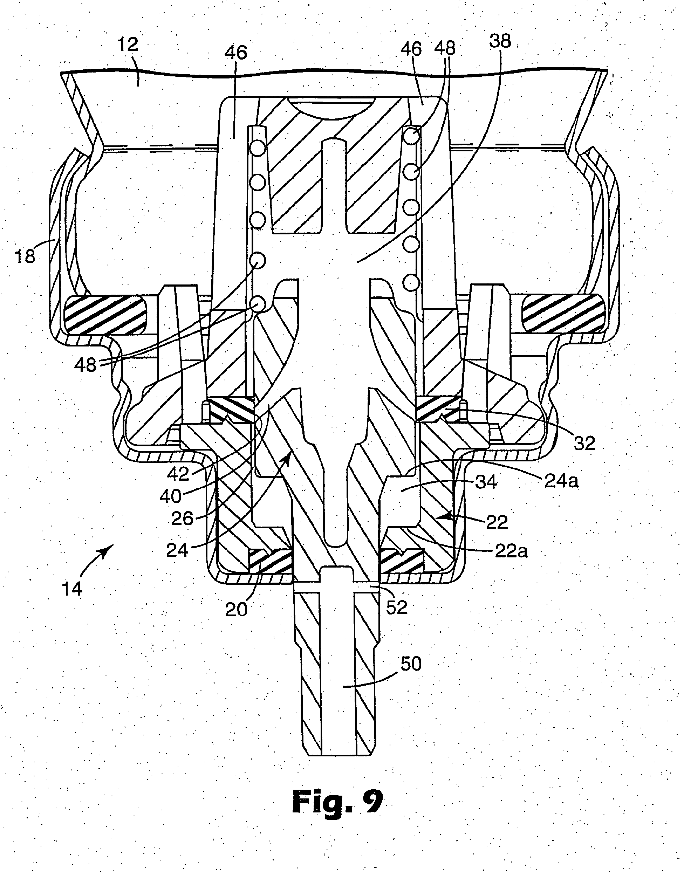

The filled stage, shown in FIG. 9, is characterized by the metering gasket **32** obstructing the flow path, thereby terminating the flow of formulation from the aerosol container **12** to the metering chamber **34.** In the metering valve design shown in FIGS. 2-5, the metering gasket **32** terminates the flow of formulation by providing sealing engagement with the portion of the valve stem side wall **24b** located between the passage opening **42** and the stem portion of the valve stem. In the metering valve design shown in FIGS. 6, 7a and 7b, the metering gasket **32** terminates flow of formulation by forming a seal with the sealing surface **44** of the valve stem **24**, as shown in FIG. 7b. In each design, the metering gasket **32** forms and maintains a fluid seal around the valve stem **24** even as the valve stem **24** continues to be displaced inwardly with respect to the metering gasket **32.** Maintenance of this fluid seal prevents any additional flow of formulation into the metering chamber **34** so that filling of the metering chamber **34** is concluded. At this stage, the metered dose of formulation is isolated and ready for discharge from the metering chamber **34** and delivery to the patient. The dimensions of the valve body **22**, valve stem **24** and other valve components determine the volume of the metering chamber **34** in the filled stage of operation.

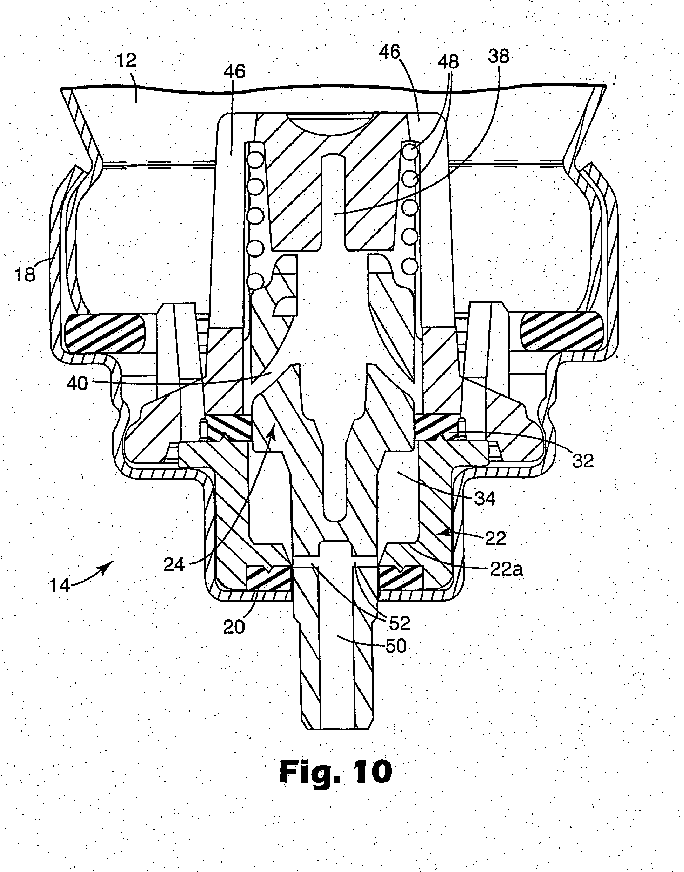

As the valve stem **24** is further displaced inwardly during actuation, the metering valve progresses to the discharge stage of operation, shown in FIG. 10. As the valve stem **24** is further actuated, the one or more side holes **52** of the discharge outlet **50** pass through the diaphragm **20** and come into fluid communication with the metering chamber **34.** That fluid communication allows the aerosol formulation within the metering chamber **34** to be released into the one or more side holes **52** and the formulation thus passes through the discharge outlet **50,** thereby delivering the metered dose of aerosol formulation to the patient or other desired area.

During the discharge of the aerosol formulation from the metering chamber **34,** the seal between the valve stem **24** and the metering gasket **32** continues to prevent the passage of additional bulk formulation from the aerosol container **12** to the metering chamber **34**.

After the dose of aerosol formulation is discharged, the valve stem **24** is returned to the original resting position. The valve stem **24** may be returned to the resting position by the biasing action of the spring **48,** or, alternatively, by some other means such as manipulation by the patient.

The successive stages of valve stem actuation are all accomplished during the brief duration of actuation of the valve stem **24.** Accordingly, formation, filling and emptying of the metering chamber **34** occurs rapidly. Only a small percentage of a dose of formulation resides in the metering chamber **34** between discharges, and the metering chamber **34** is full of formulation only for a brief moment immediately prior to discharge of the formulation from the metering chamber **34.** Subsequent release of the valve stem by the patient allows the valve to return from the position of the valve stem **24** during the discharge stage to the resting position. During the return of the valve stem **24** to the resting position, formulation may enter the metering chamber **34** as soon as the metering gasket **32** no longer occludes the flow path into the metering chamber **34.** While cavitation of formulation may occur at this time, subsequent travel of the valve stem **24** as it completes its return to the resting position causes the formulation to be returned to the interior chamber **38** via the annular gap **26.** An annular gap having a short length helps to ensure purging of the residual vapor and ease of return of the valve stem **24** against the force due to viscous drag.

Various modifications and alterations to this invention will become apparent to those skilled in the art without departing from the scope and spirit of this invention. It should be understood that this invention is not intended to be unduly limited by the illustrative embodiments and examples set forth herein and that such examples and embodiments are presented by way of example only with the scope of the invention intended to be limited only by the claims set forth herein as follows.

**Claims**

**1.**    An aerosol metering valve comprising:

a body wall defining an internal chamber and comprising a metering gasket and a diaphragm, the diaphragm having walls defining an aperture;

a valve stem comprising a body portion and a stem portion, the body portion comprising a bottom edge and being positioned in the internal chamber of the metering valve, the stem portion comprising a discharge outlet and passing through the aperture in slidable, sealing engagement with the diaphragm;

an annular gap comprising an inlet and having a width defined by the distance from the body wall to the valve stem, and also having a length defined by the distance from the inlet to the bottom edge of the body portion of the valve stem, and wherein a ratio of the length of the annular gap to the width of the annular gap is less than about 40:1; and a flow path providing at least transient fluid communication between the internal chamber and the annular gap through the inlet.

2. The aerosol metering valve of claim 1 wherein the flow path comprises a -passageway through the body portion of the valve stem.

3. The aerosol metering valve of claim 1 wherein the flow path comprises a clearance between the body portion of the valve stem and the metering gasket.

4. The aerosol metering valve of claim 1 wherein the ratio of the length of the annular gap to the width of the annular gap is less than about 25:1.

5. The aerosol metering valve of claim 1 wherein the ratio of the length of the annular gap to the width of the annular gap is less than about 20:1.

6. The aerosol metering valve of claim 1 wherein the ratio of the length of the annular gap to the width of the annular gap is less than about 5:1.

7. The aerosol metering valve of claim 1 wherein the ratio of the length of the annular gap to the width of the annular gap is less than about 1:1.

**Fig. 1**

**Fig. 2**

**Fig. 3**

**Fig. 4**

**Fig. 5**

**Fig. 6**

**Fig. 7a**

**Fig. 7b**

Fig. 8

**Fig. 9**

**Fig. 10**